# EUROPÄISCHE PATENTSCHRIFT

(11) **EP 0 580 556 B1**
(45) Veröffentlichungstag und Bekanntmachung des Hinweises auf die Patenterteilung: **29.01.1997**
(21) Anmeldenummer: 93810521.0
(22) Anmeldetag: 21.07.1993
(51) Int. Cl.: A61F 9/02, A61F 9/06

(54) **Einrichtung zum Schutz des menschlichen Kopfes gegen äussere Einwirkungen**
Device for the protection of the human head against external elements
Dispositif protecteur de la tête humaine contre des éléments extérieurs

(30) Priorität: 24.07.1992 DE 4224476
(43) Veröffentlichungstag der Anmeldung: 26.01.1994
(73) Patentinhaber: OPTREL AG, 9630 Wattwil (CH)
(72) Erfinder: Del Bon, Francesco, CH-4663 Aarburg (CH); Lutz, Christoph, CH-8124 Maur (CH)
(74) Vertreter: Rottmann, Maximilian R.

(56) Entgegenhaltungen:
- AU-A- 2 400 277
- NL-C- 49 498
- US-A- 2 398 269
- US-A- 3 075 201
- US-A- 3 214 768
- US-A- 4 464 800
- US-A- 4 536 892

## Beschreibung

Die vorliegende Erfindung betrifft eine Einrichtung zum Schutz des menschlichen Kopfes gegen äussere Einwirkungen, mit einer am Kopf des Trägers der Schutzeinrichtung zu befestigenden Tragvorrichtung und einem die Schutzfunktion ausübenden Visier, welches mit der Tragvorrichtung aus der Gebrauchslage hochschwenkbar verbunden ist, und mit Einstellmitteln zum Verstellen und Fixieren der Lage des Visiers in Sehrichtung und der unteren Schwenkendlage des Visiers.

Bei einer bekannten derartigen Schutzeinrichtung in Form eines Schutzschildes für Schweissarbeiten umfasst die Tragvorrichtung ein in seiner Länge verstellbares Stirnband und einen das Stirnband mit radialem Abstand umgebenden Tragring mit hochstehendem Bandprofil, welcher zur Erhöhung der Formstabilität mit einem Kragen versehen ist und an welchem das Stirnband und zwei sich kreuzende textile Kopfbänder sowie das Visier befestigt sind. Für die Befestigung dieser Teile am Tragring sind gebrauchsmässig nicht lösbare Steckverbindungen vorgesehen, wobei zwischen Stirnband und Tragring als zusätzliche Verbindungsmittel radial nachgiebige Laschen eingesetzt sind. Seitlich am Tragring sind Lagerstücke angesetzt, in denen je ein seitlich abstehender Gelenkbolzen für die Schwenklagerung des Visiers verankert ist. Das Visier weist Seitenwangen auf, in welchen sich Durchgangsöffnungen für die Gelenkbolzen befinden. Als Verbindungsmittel zwischen Visier und Gelenkbolzen dienen Rutschkupplungen, deren Reibungswiderstand durch aussen am Visier betätigbare Stellmuttern einstellbar ist. Tragring und Stirnband sowie sämtliche Zubehörteile, mit Ausnahme der Teile für die Gelenkverbindung, sind aus Kunststoff hergestellt.

Die Durchgangsöffnungen in den Seitenwangen des Visiers sind als Langlöcher ausgebildet, welche in der Gebrauchslage des Visiers etwa horizontal verlaufen, so dass das Visier in Sehrichtung verstellbar und den Bedürfnissen des Trägers anpassbar ist.

Ferner ist eine Anschlagvorrichtung zur Festlegung der Gebrauchslage, d.h. der unteren Schwenkendlage des Visiers vorhanden, wobei eine Seitenwange des Visiers an ihrer Innenseite eine zur Längsachse des Langlochs parallel verlaufende Anschlagleiste und der Tragring einen mit der Anschlagleiste zusammenarbeitenden verstellbaren Anschlag aufweist. Der letztere befindet sich am Ende des einen Arms eines zweiarmigen Hebels, dessen zweiter Hebelarm als Zahnsegment ausgebildet ist.

Der zweiarmige Hebel ist zwischen der Seitenwange des Visiers und dem Tragring auf dem diesseitigen Schraubenbolzen schwenkbar gelagert, und das Zahnsegment arbeitet mit einem entsprechend verzahnten Ansatz am betreffenden Lagerstück zusammen. Durch Verändern der gegenseitigen Eingriffslage zwischen den Zähnen des Zahnsegments und denjenigen des verzahnten Ansatzes lässt sich dieser Anschlag und damit der Neigungswinkel des Visiers einstellen. Das Zahnsegment ist elastisch nachgiebig ausgebildet und kann zum Verstellen des Anschlags durch Wegbiegen vorübergehend vom Ansatz getrennt, d.h. mit der Verzahnung desselben ausser Eingriff gebracht werden. Durch die Parallelität der Anschlagleiste bezüglich der Längsachse des Langlochs ist gewährleistet, dass der eingestellte Neigungswinkel des Visiers beim Verschieben desselben in Sehrichtung erhalten bleibt.

Das Stirnband dieser Schutzeinrichtung ist aus einem steif-elastischen Bandstück gebildet, dessen Enden Verbindungsmittel zum Schliessen des Stirnbandes in jeweils wählbarer Länge aufweisen. Diese Verbindungsmittel bestehen darin, dass das eine Ende des Bandstückes eine Längsreihe von Löchern und das andere Ende einen Zapfen aufweist, welche letzterer bei übereinandergelegten Bandenden in jeweils eines der Löcher des einen Bandendes eindrückbar ist.

In der Praxis hat es sich jedoch auch gezeigt, dass insbesondere die Einstellmittel für die Fixierung der unteren Endlage des Visiers ungünstig angeordnet und praktisch nur bei abgenommener Schutzeinrichtung zu handhaben sind, so dass die Schutzeinrichtung unter Umständen schrittweise angepasst und dazu mehrmals abgenommen und wieder aufgesetzt werden muss, bis sie richtig passt. Das gleiche trifft zu für die Veränderung des Umfangs des Stirnbandes, was vor allem dann nachteilig ist, wenn die Druckempfindlichkeit am Kopf des Trägers häufig wechselt und entsprechend oft eine erneute Anpassung erfordert, oder wenn die Schutzeinrichtung häufig von verschiedenen Personen benutzt wird.

Beispielsweise ist aus der AU-A-2,400,277 eine Schutzeinrichtung bekannt, bei der Einstellmittel zum Verstellen und Fixieren der Lage des Visiers in Sehrichtung und der unteren Schwenkendlage des Visiers vorhanden sind. An der Aussenseite der Schutzeinrichtung ist eine Fixiermutter zum Feststellen der Einstellmittel angeordnet. Nach dem Lösen der Fixiermutter müssen die mit Bolzen und Nuten ineinandergreifenden Teile der Einstellmittel achsial auseinandergezogen werden, um die genannten Teile gegenseitig ausser Eingriff zu bringen, damit sie verstellt werden können. Eine solche Handhabung ist aber praktisch nicht möglich, wenn sich die Schutzeinrichtung in der Gebrauchslage befindet. Von den beiden Stellorganen befindet sich nur eines auf der Aussenseite der Schutzeinrichtung.

Ferner ist aus der US-A-3,075,201 eine Schutzeinrichtung bekannt, bei der nur die untere Schwenkendlage des Visiers verstellbar ist, wobei ein Hebel als Einstellmittel an der Innenseite des Visiers angeordnet ist. An der Aussenseite der Schutzeinrichtung befinden sich keine diesbezüglichen Einstellmittel.

In der US-A-3,214,768 sie liegt dem Oberbegriff des Anspruchs 1 Zugrunde ist eine mit Tragband und Visier ausgerüstete Schutzeinrichtung beschrieben, welche wahlweise auf barem Kopf oder über einer Schutzkappe getragen wird. Das Visier ist um einen Drehpunkt an einem Haltebügel schwenkbar gelagert, der seinerseits fest mit der Tragvorrichtung verbunden ist. Verstellmittel der im Anspruch der Anmeldung genannten Art sind bei dieser Schutzvorrichtung nicht vorhanden. Die von aussen zugängliche Schraubenmutter dient einzig dazu, die Klemm- bzw. Reibungskraft zwischen Visier und Haltebügel einzustellen.

Ein Hakenhebel, welcher aus einer horizontalen in eine vertikale Lage schwenkbar ist, dient beim Tragen der Schutzvorrichtung über einer Schutzkappe zum Festhalten der Tragvorrichtung an der Schutzkappe, indem der in diesem Fall vertikal gestellte Hakenhebel mit seinem Haken den unteren Rand der Schutzkappe untergreift.

Schliesslich ist aus der US-A-4,464,800 eine Schutzeinrichtung mit einem Adapter zur Montage eines Visiers an Tragvorrichtungen verschiedener Hersteller bekannt. Diese Schutzeinrichtung weist jedoch keine Mittel auf, die es ermöglichten, das Visier mit von aussen bedienbaren Einstellmitteln manuell zu verstellen.

Der Erfindung liegt daher die Aufgabe zugrunde, eine Schutzeinrichtung der eingangs genannten Art so zu verbessern, dass sie bei einfachem konstruktivem Aufbau in deren Gebrauchslage am Kopf in jeder Beziehung vom Träger angepasst werden kann.

Diese Aufgabe wird erfindungsgemäss durch die im Kennzeichen des Anspruchs 1 angegebenen Merkmale gelöst.

Dadurch, dass das Visier unmittelbar am Stirnband angelenkt ist, ergeben sich einfache konstruktive Voraussetzungen. Ausgehend von der bekannten Lösung, bei der das Visier Seitenwangen aufweist, welche an der Tragvorrichtung angelenkt und durch je eine lösbare, als Rutschkupplung arbeitende Schraubverbindung an dieser gehalten sind, kann unter diesem Umstand die Anordnung so getroffen sein, dass an dem Stirnband in dessen Schläfenpartien je ein radial nach aussen abgesetzter Bandabschnitt gebildet und als Basisteil für die Festlegung der Schraubverbindung ausgebildet ist. Dabei kann der abgesetzte Bandabschnitt und die diesen überdeckende Seitenwange des Visiers je eine Durchgangsöffnung für einen die Schwenkachse des Visiers bildenden Schraubenbolzen aufweisen, wobei eine der beiden Durchgangsöffnungen als Langloch ausgebildet ist, welches wenigstens annähernd parallel zur Sehrichtung verläuft.

Bei der vorerwähnten bekannten Schutzeinrichtung sind an den Aussenseiten der Seitenwangen des Visiers im Bereich der entsprechenden Langlöcher Rastkerben vorgesehen, in welche entsprechend ausgebildete Rippen einer durch die Stellmutter festklemmbaren Unterlegscheibe eingreifen, so dass die Gelenkbolzen des Visiers an bestimmten Raststellen fixierbar sind. Allerdings handelt es sich dabei um eine Mehrzahl von Raststellen, was in der Regel dazu führt, dass die beiden Gelenkbolzen eher zufällig beidseitig die gleiche Stellung einnehmen und demzufolge das Visier meistens mit einer seitlichen Abweichung seiner Längsachse inbezug auf die Sehrichtung an der Tragvorrichtung fixiert wird, insbesondere beim Einstellen des Visiers in der Gebrauchslage der Schutzeinrichtung.

Eine in dieser Beziehung verbesserte Lösung besteht bei der erfindungsgemässen Schutzeinrichtung darin, dass das Langloch in der Längsrichtung einige, beispielsweise drei Raststellen zur wahlweisen Fixierung des Schraubenbolzens durch Festziehen der Schraubverbindung mit Hilfe eines ausserhalb der Seitenwange des Visiers auf den Schraubenbolzen aufgesetzten Stellknopfes aufweist. Diese Lösung mit wenigen Raststellen bietet Gewähr für eine stets geradlinige Einstellung des Visiers.

Vorzugsweise befindet sich die als Langloch ausgebildete Durchgangsöffnung in den abgesetzten Bandabschnitten des Stirnbandes, und nicht wie bei der bekannten Ausführung in den Seitenwangen des Visiers. Unter Berücksichtigung des Umstandes, dass die Bedienungselemente jeweils nur einen Teil der Langlöcher verdecken, werden auf diese Weise unbedeckte, das Eindringen von Fremdkörpern ermöglichende Öffnungen in dem als Schutzschild dienenden Visier vermieden.

Zum Fixieren der unteren Schwenkendlage des Visiers ist in an sich bekannter Weise eine Anschlagvorrichtung vorgesehen, welche aus einem festen Anschlag und einem mit diesem zusammenarbeitenden verstellbaren Anschlagorgan besteht. Mit Vorteil ist dabei die Anordnung im Sinne der Erfindung so getroffen, dass der feste Anschlag an einem der abgesetzten Bandabschnitte des Stirnbandes und das verstellbare Anschlagorgan an der Innenseite der diesem Bandabschnitt zugewandten Seite der betreffenden Seitenwange des Visiers angeordnet ist. Im Rahmen dieser generellen Anordnung sind zahlreiche konstruktive Lösungen möglich. Hervorzuheben ist dabei die Lösung, wonach sich die Anschlagvorrichtung und deren Einstellmittel ausserhalb der Ohrenpartie der Tragvorrichtung befinden. Diese Anordnung ermöglicht es, an den abgesetzten Bandabschnitten des Stirnbandes zusätzlich Ohrenschützer anzubringen, ohne dabei die Funktion der Anschlagvorrichtung zu behindern.

In den beiliegenden Zeichnungen sind Ausführungsbeispiele der erfindungsgemässen Einrichtung zum Schutz des menschlichen Kopfs dargestellt, und zwar zeigen:
- Fig. 1: eine Ansicht eines die Tragvorrichtung bildenden Traggerüstes für die erfindungsgemässe Schutzeinrichtung;
- Fig. 2: eine Abwicklung der unter sich zusammenhängenden, das Traggerüst nach Fig. 1 bildenden Bandstücke;
- Fig. 3: eine Einzelheit der am Stirnband vorgesehenen Schliesse;
- Fig. 4: eine Ansicht einer kompletten Schutzeinrichtung mit einer das Traggerüst nach Fig. 1 enthaltenden Tragvorrichtung;
- Fig. 5: ein Visier der Schutzeinrichtung nach Fig. 4, von der anderen Seite gesehen;
- Fig. 6: Teile der Verbindung zwischen Traggerüst und Visier, in Explosionsdarstellung;
- Fig. 7: eine Detailansicht einer Anschlagvorrichtung für ein aufklappbares Visier;
- Fig. 8: eine Ansicht einer inbezug auf die Längsverstellung des Visiers abgeänderten Ausführungsform der Schutzeinrichtung, in demontiertem Zustand;
- Fig. 9: die Schutzeinrichtung nach Fig. 7, in montiertem Zustand;
- Fig. 10: eine Ansicht des Visiers mit einer anderen Ausführungsform des verstellbaren Anschlagorgans;
- Fig. 11: eine Ansicht der kompletten Schutzeinrichtung mit dem verstellbaren Anschlagorgan nach Fig. 9;
- Fig. 12: eine Ansicht des Visiers mit einer weiteren Ausführungsform des verstellbaren Anschlagorgans;
- Fig. 13: eine Aussenansicht der kompletten Schutzeinrichtung mit einem verstellbaren Anschlagorgan nach Fig. 11;
- Fig. 14: eine Ansicht der Tragvorrichtung mit einem festen Anschlag gemass einer weiteren Ausführungsform der Anschlagvorrichtung;
- Fig. 15: eine Teilansicht der gesamten Anschlagvorrichtung mit der Tragvorrichtung nach Fig. 13;
- Fig. 16: Einstellmittel für die Anschlagvorrichtung mach Fig. 15, teilweise im Achsialschnitt;
- Fig. 17: eine Ansicht der Tragvorrichtung mit einem festen Anschlag gemäss einer weiteren Ausführungsform der Anschlagvorrichtung; und
- Fig. 18: eine Teilansicht der gesamten Anschlagvorrichtung mit der Tragvorrichtung nach Fig. 13.

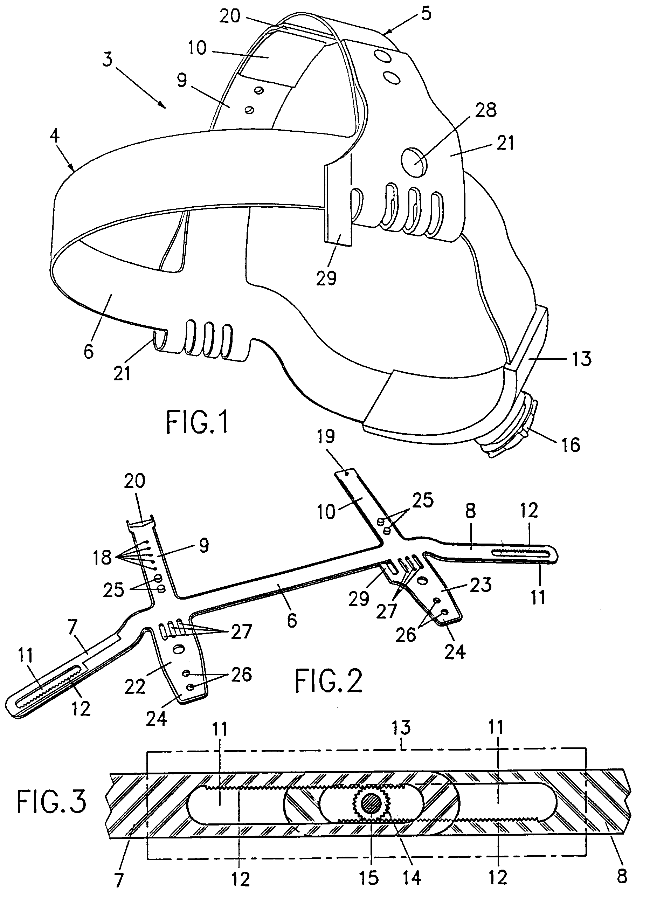

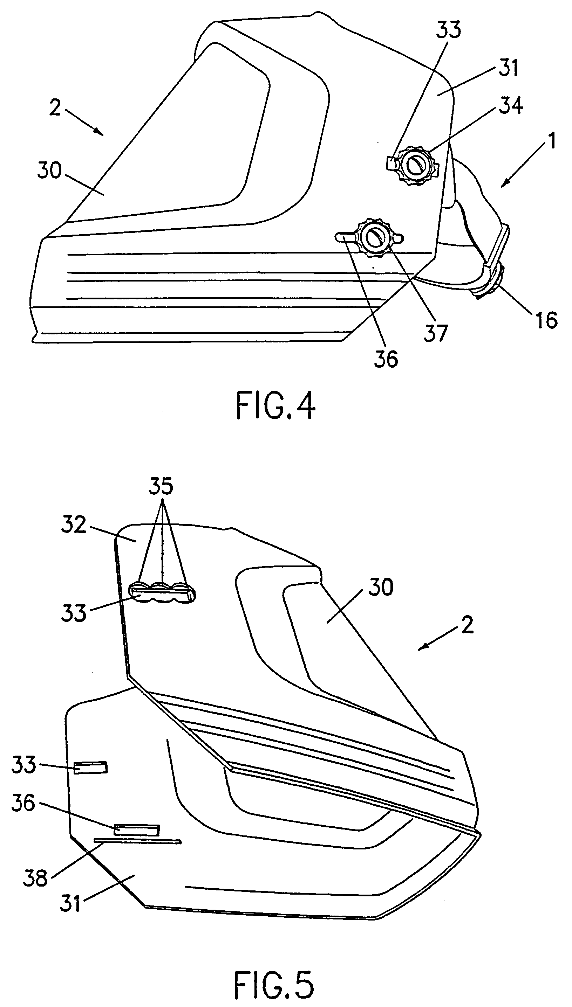

Die in Fig. 4 gesamthaft dargestellte Schutzeinrichtung besteht im wesentlichen aus einer Tragvorrichtung 1 und einem daran befestigten aufklappbaren Visier 2. Die Tragvorrichtung 1 wird ihrerseits zur Hauptsache durch ein Traggerüst 3 gebildet, welches in Fig. 1 im aufgebauten Zustand dargestellt ist.

Das Traggerüst 3 nach Fig. 1 ist aus kompaktem Material, vorzugsweise aus Kunststoff, wie z.B. Polyäthylen, einstückig ausgebildet und umfasst ein Stirnband 4 und ein zum Stirnband quer verlaufendes Kreuzband 5. Das in Fig. 2 dargestellte Ausgangsprodukt zum Aufbau des Traggerüsts 3 ist ein flaches Gebilde mit steif-elastischen Bandstücken, wobei ein längeres Bandstück 6 durch Rundbiegen und Zusammenfügen seiner beiden Enden 7 und 8 das ringförmige Stirnband 4 bildet und zwei vom Bandstück 6 quer abstehende Bandstücke 9 und 10 durch anschliessendes Biegen und Zusammenfügen ihrer freien Enden 11 bzw. 12 das Kreuzband bilden. Wie in Fig. 2 angedeutet ist, sind die Bandstücke 6, 9 und 10 an den Kanten verstärkt.

An den Enden der Bandstücke sind Verbindungsmittel zum Schliessen des Stirnbandes 4 und des Kreuzbandes 5 in jeweils wählbarer Länge derselben vorgesehen. Diese Verbindungsmittel bestehen beim Stirnband 4 darin, dass die freien Enden 7 und 8 des das Stirnband 4 bildenden Bandstücks 6 je ein in Bandlängsrichtung verlaufendes Langloch 11 aufweisen, dessen eine Längskante mit einer Verzahnung 12 versehen ist. Dabei liegen die Verzahnungen 12 der beiden Langlöcher 11 auf verschiedenen Längsseiten der Langlöcher, so dass sie bei übereinandergelegten Bandenden 7, 8 einander gegenüberliegen (Fig. 3).

Ferner ist eine die beiden, die Nackenpartie des Stirnbandes 4 bildenden Bandenden 7, 8 umfassende Schliesse 13 vorgesehen ist, welche im Innern ein in die Verzahnungen 12 der beiden Bandenden 7, 8 eingreifendes Zahnrad 14 sowie eine die Drehung des Zahnrades hemmende Rutschkupplung (nicht dargestellt) und aussen einen mit der Achse 15 des Zahnrads 14 verbundenen Drehknopf 16 aufweist. Durch Drehen des Drehknopfes 16 werden die beiden Bandenden 7, 8 je nach Drehrichtung im Sinne einer Verkürzung oder einer Verlängerung des Stirnbandes 4 gegeneinander stufenlos verschoben, wobei die eingestellte Länge infolge der Rutschkupplung jeweils erhalten bleibt. Ohne Betätigung des Drehknopfs 16 gibt die Rutschkupplung erst dann nach, wenn äussere Kräfte auf das Stirnband 4 einwirken und diese eine bestimmte Sicherheitsgrenze überschreiten.

Zum Schliessen des Kreuzbandes 5 weist das eine Bandstück 9 desselben eine Längsreihe von Löchern 18 und das andere Bandstück 10 an seinem freien Ende einen Zapfen 19 auf, welch letzterer bei übereinandergelegten Bandstücken 9, 10 in jeweils eines der Löcher 18 des Bandstücks 9 eindrückbar ist. Damit sich dieser Verschluss nicht ohne weiteres lösen kann, ist das freie Ende des Bandstücks 9 mit einer Schlaufe 20 versehen, welche das andere Bandstück 10 umfasst.

Ferner weist das Stirnband 4 zwei radial nach aussen abgesetzte Bandabschnitte 21 auf, welche sich an den Schläfenseiten des Stirnbandes 4 befinden und als Verbindungsteile für die Befestigung des Visiers 2 ausgebildet sind. Im vorliegenden Beispiel sind die genannten Bandabschnitte 21 je aus einem weiteren Bandstück 22 bzw. 23 gebildet, welches von dem das Stirnband 4 bildenden Bandstück 6 quer absteht und durch Umbiegen nach aussen und nach oben zu einer Schlaufe geformt ist. Die Ansatzstellen der die abgesetzten Bandabschnitte 21 bildenden Bandstücke 22, 23 und der das Kreuzband 5 bildenden Bandstücke 9, 10 befinden sich an einander gegenüberliegenden Seitenkanten des Stirnbandes 4, und die Enden 24 der Bandstücke 22, 23 sind an den das Kreuzband 5 bildenden Bandstücken 9 bzw. 10 befestigt, wozu in den Bandstücken 9, 10 je zwei Zapfen 25 und in den Bandstücken 22, 23 zwei diese Zapfen 25 aufnehmende Löcher 26 vorgesehen sind. Im Biegebereich ist der Querschnitt der Bandstücke 22, 23 durch Aussparungen 27 vermindert.

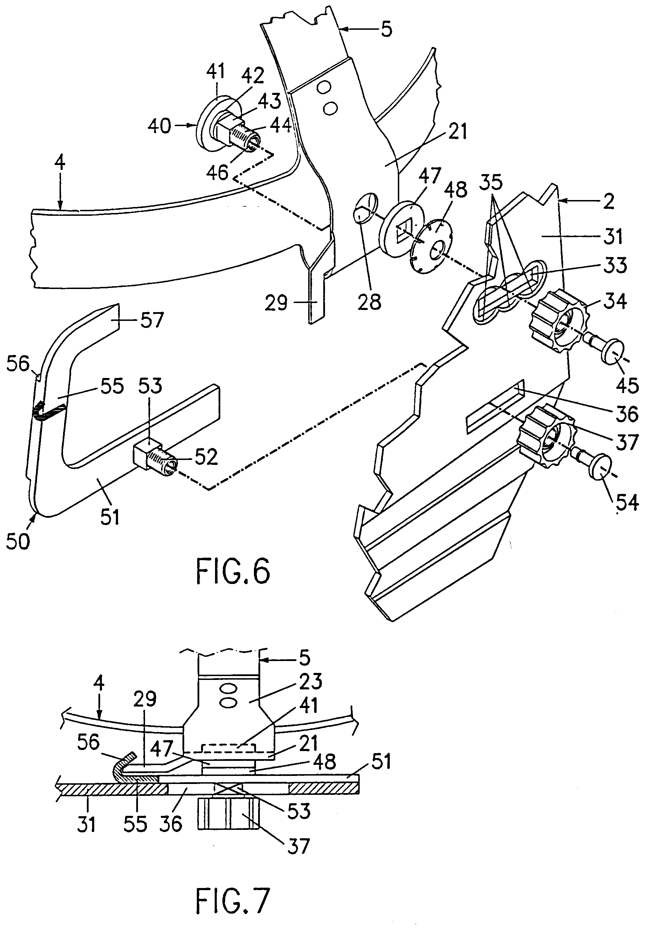

Jeder abgesetzte Bandabschnitt 21 weist eine Durchgangsöffnung 28 für den Einsatz der Verbindungselemente auf, welche zur Befestigung des Visiers 2 am Traggerüst 3 dienen. Ferner ist am Bandstück 23 ein Anschlag 29 angeformt, welcher die untere Schwenkendlage, d.h. die Gebrauchslage des Visiers 2 fixiert. Die genannten Verbindungsmittel und Anschlagmittel werden später anhand der Fig. 6 und 7 im einzelnen erläutert.

Das beschriebene Traggerüst 3 nach Fig. 1 ist in der Regel noch mit einer hier nicht dargestellten Polsterung versehen, welche an der Innenseite des Stirnbandes 4 angebracht ist, sich über dessen Länge mit Ausnahme der Enden 7 und 8 erstreckt und auch Teile des Kreuzbandes 5 bedecken kann.

Anstelle der zu Schlaufen gebogenen Bandstücke 22, 23 können die abgesetzten Bandabschnitte auch durch am Stirnband angeformte Schlaufen gebildet sein. Ferner können die abgesetzten Bandabschnitte durch formstabile Ausbuchtungen am Stirnband 4 ausgebildet sein.

Gemäss den Fig. 4 und 5 besitzt das an der Tragvorrichtung 1 montierte Visier 2 an der Frontseite ein Fenster 30, das beispielsweise durch eine Filterscheibe mit durch die Helligkeit des einfallenden Lichts gesteuerter Transparenz abgeschlossen ist, und zwei sich nach hinten erstreckende Seitenwangen 31 und 32, welche mit dem Traggerüst 3 gelenkig verbunden sind. In beiden Seitenwangen 31, 32 befindet sich je ein Längsschlitz 33, durch den sich ein Gelenkbolzen (hier nicht sichtbar) nach aussen erstreckt, welcher in die Durchgangsöffnung 28 der abgesetzten Bandabschnitte 21 des Traggerüsts 3 eingesetzt ist. Der beidseitige Längsschlitz 33 ermöglicht eine Verschiebung des Visiers 2 in Sehrichtung. Auf dem mit Gewinde versehenen Gelenkbolzen sitzt eine mit Drehknopf 34 versehene Stellmutter zum Fixieren des Visiers in der eingestellten Verschiebelage. Im vorliegenden Beispiel sind in der Verschieberichtung drei Raststellen vorgesehen und durch an der Aussenseite der Seitenwangen 31, 32 eingeformte Vertiefungen 35 festgelegt, in welche beim Anziehen der Stellmutter ein entsprechender Rastansatz am Drehknopf 34 eingreift. Zur Einstellung der unteren Schwenkendlage bzw. Gebrauchslage des aufklappbaren Visiers 2 ist ein in den Fig. 4 und 5 nicht dargestellter verschiebbarer Anschlag vorgesehen, welcher mit dem Anschlag 29 am Traggerüst 3 (Fig. 1) zusammenarbeitet und welcher mit einem Schraubenbolzen den an der linken Seitenwange 31 des Visiers 2 vorhandenen Längsschlitz 36 durchsetzt und durch eine mit Drehknopf 37 versehene Stellmutter fixierbar ist. Unterhalb dieses Längsschlitzes 36 ist an der Innenseite der Seitenwange 31 eine Leiste 38 zur Geradführung des genannten Anschlags angeformt. Weitere Einzelheiten werden nachstehend anhand der Fig. 6 und 7 beschrieben.

In den Fig. 6 und 7 sind für übereinstimmende Teile die gleichen Bezugszeichen verwendet wie in den vorher beschriebenen Fig. 1 bis 5. In der Explosionsdarstellung gemäss Fig. 6 erkennt man die Teile der Gelenkverbindung zwischen dem Visier 2 und dem Traggerüst 3. Die Schwenkachse bildet ein Gelenkbolzen 40, welcher mit einem Kopf 41, einem zylindrischen Ansatz 42, einem Vierkantteil 43 und an seinem Ende mit einem Gewindeteil 44 versehen ist. Der Gelenkbolzen 40 ist mit seinem zylindrischen Ansatz 42 in der kreisrunden Durchgangsöffnung 28 des vom Stirnband 4 abgesetzten Bandabschnittes 21 drehbar gelagert, wobei sich der Kopf 41 des Gelenkbolzens 40 auf der dem Stirnband 4 zugewandten Seite des Bandabschnitts 21 befindet. Der Vierkantteil 43 des Gelenkbolzens 40 ist in dem rechteckigen Längsschlitz 33 längsverschiebbar und unverdrehbar geführt. Auf den Gewindeteil 44 ist der Drehknopf 34 mit der in diesem verankerten Stellmutter aufgeschraubt und durch einen Sperrstift 45, welcher in eine Zentralbohrung 46 des Gelenkbolzens 40 eingreift, in an sich bekannter Weise gegen Verlieren gesichert. Zwischen dem abgesetzten Bandabschnitt 21 und der Seitenwange 31 des Visiers 2 befinden sich auf dem Gelenkbolzen 40 eine Zwischenscheibe 47 mit Vierkantloch und eine Federscheibe 48. Letztere hält den in eine der Vertiefungen 35 eingedrehten Drehknopf 34 während der Schwenkbewegung des Visiers 2 an dieser Raststelle fest.

Zur Fixierung der unteren Schwenkendlage, d.h. der Gebrauchslage des Visiers 2, ist eine stufenlos verstellbare Anschlagvorrichtung vorgesehen, welche den bereits beschriebenen festen Anschlag 29 und ein verschiebbares und in der gewünschten Lage feststellbares Anschlagorgan 50 umfasst. Im vorliegenden Beispiel befindet sich die Anschlagvorrichtung an der linken Seite des Visiers 2; selbstverständlich könnte sie auch an der rechten Seite desselben angeordnet sein. Das Anschlagorgan 50 hat die Form eines flachen Bügels und besitzt einen unteren, geradlinigen Schenkel 51, welcher sich auf der Führungsleiste 38 (Fig. 5) abstützt und einen festsitzenden Gewindebolzen 52 mit Vierkantteil 53 aufweist. Der Vierkantteil 53 ist in dem rechteckigen Längsschlitz 36 in der Seitenwange 31 des Visiers 2 längsverschiebbar und unverdrehbar geführt. Auf den Gewindebolzen 52 ist der Drehknopf 37 mit einer darin verankerten Stellmutter aufgeschraubt und in gleicher Weise wie der Drehknopf 34 mit einem Sperrstift 54 gesichert. Ein quer zum Schenkel 51 verlaufender Schenkel 55 des Anschlagorgans 50 weist einen zu einem Haken 56 umgebogenen Lappen auf, welcher als diesseitiger Anschlagteil mit dem feststehenden Anschlag 29 in der in Fig. 7 gezeigten Weise zusammenarbeitet. Ein weiterer, oberer Schenkel 57, welcher etwa parallel zum unteren Schenkel 51 verläuft, dient dazu, bei der Schwenkbewegung des Visiers 2 aus der hochgeklappten Ruhelage in die Gebrauchslage den abgesetzten Bandabschnitt 21 zu hintergreifen und dabei den Haken 56 mit seiner Öffnung an den festen Anschlag 29 heranzuführen.

Die Länge des Schenkels 51 des verstellbaren Anschlagorgans 50 ist vorzugsweise so bemessen, dass der Schenkel 51 in jeder Verschiebelage des Anschlagorgans 50 den Längsschlitz 36 in der Seitenwange 31 des Visiers 2 abdeckt, um die Schutzfunktion des Visiers an dieser Stelle nicht durch eine sonst freie Öffnung zu beeinträchtigen.

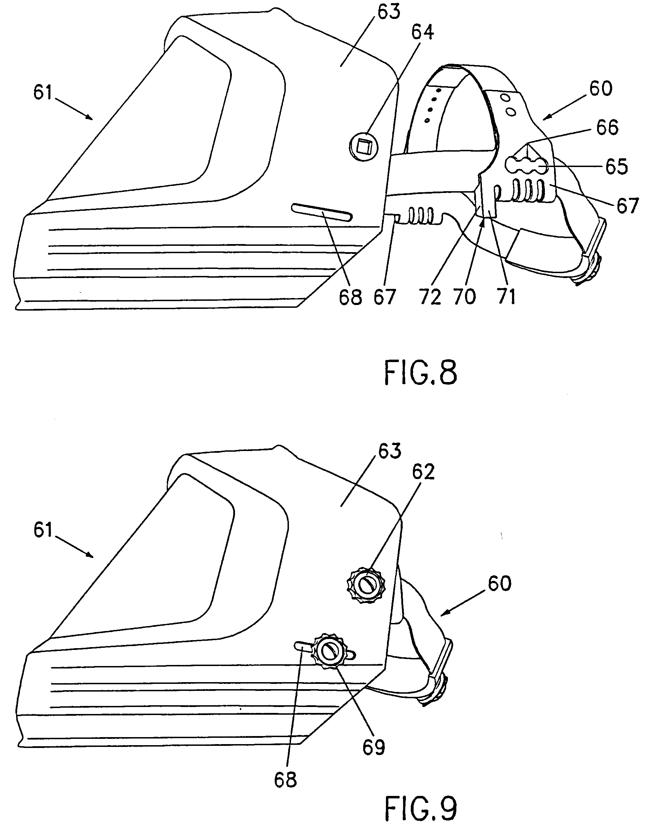

Die Ausführungsform nach den Fig. 8 und 9, welche ebenfalls eine Tragvorrichtung 60 und ein dazugehöriges Visier 61 zeigen, unterscheidet sich von derjenigen nach den Fig. 4 und 5 dadurch, dass der mit dem Stellknopf 62 fixierbare Gelenkbolzen jeweils in der Seitenwange 63 des Visiers 61 mit einer Lagerbüchse 64 festgelegt ist, während sich der für die Verschiebung des Visiers 61 in Sehrichtung vorgesehene Längsschlitz 65 mit den Raststellen 66 nunmehr in den abgesetzten Bandabschnitten 67 der Tragvorrichtung 60 befindet. Auf diese Weise entfällt ein entsprechender Längsschlitz in den Seitenwangen des Visiers, womit die damit verbundene Gefahr einer Beeinträchtigung der Schutzwirkung vermieden wird. Der Längsschlitz 68 für die Verschiebung des verstellbaren Anschlagorgans mit dem Stellknopf 69 ist in dieser Beziehung weniger kritisch, wie anhand der Fig. 7 gezeigt worden ist. Das hier nicht dargestellte verstellbare Anschlagorgan arbeitet mit einem festen Anschlag 70 zusammen, welcher einen am diesseitigen abgesetzten Bandabschnitt 67 der Tragvorrichtung 60 angeformten Lappen 71 und einen von der Längskante desselben radial nach innen abstehenden Steg 72 aufweist.

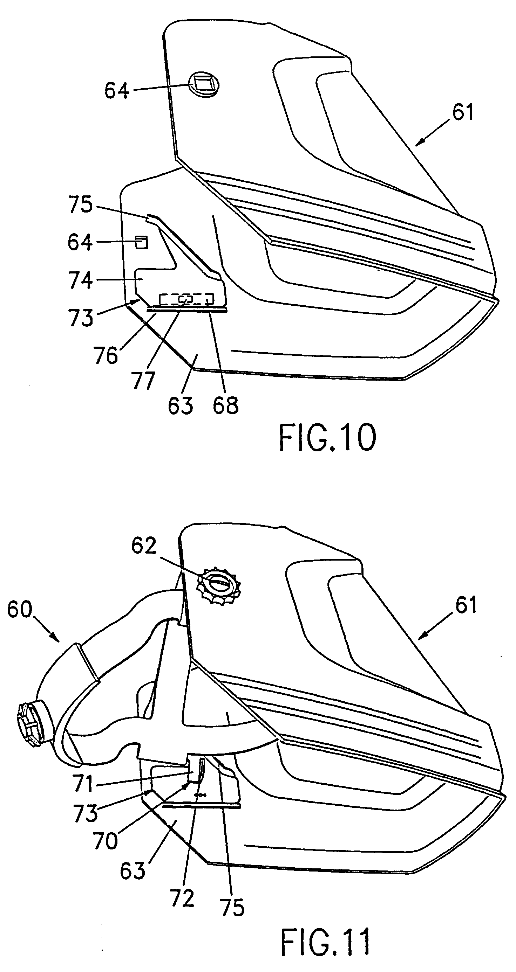

Eine ähnliche Ausführungsform, aber mit einer Anschlagvorrichtung mit einem gegenüber den Fig. 6 und 7 abgeändertem verstellbaren Anschlagorgan, ist in den Fig. 10 und 11 dargestellt. Die übrigen Teile stimmen mit denjenigen der Fig. 8 und 9 überein und sind mit den gleichen Bezugszeichen versehen. Das verstellbare Anschlagorgan 73 besteht aus einem plattenförmigen Teil 74 und einer von dieser senkrecht abstehenden Anschlagkante 75, welch letztere mit dem festen Anschlag 70 zusammenarbeitet. Der plattenförmige Teil 74 ist in gleicher Weise wie bei der Ausführungsform nach den Fig. 6 und 7 längsverschiebbar geführt, wozu die Seitenwange 63 des Visiers ausser dem Längsschlitz 68 eine Führungsleiste 76 aufweist. Der am plattenförmigen Teil 74 befestigte, aussen mit dem Stellknopf 69 (Fig. 9) versehene Schraubenbolzen zur Fixierung des verstellbaren Anschlagorgans 73 ist bei 77 angedeutet.

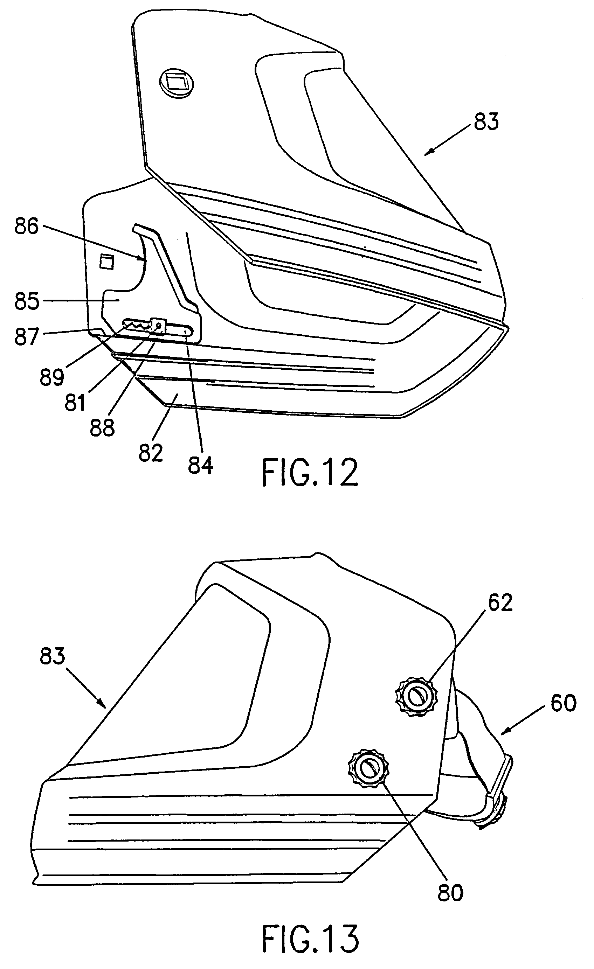

Um den Längsschlitz in der Seitenwange des Visiers zur Verschiebung des Schraubenbolzens des verstellbaren Anschlagorgans zu vermeiden, kann eine Lösung gemäss den Fig. 12 und 13 gewählt werden, bei welcher die Lage des mit dem Stellknopf 80 versehenen Schraubenbolzens 81 in der Seitenwange 82 des Visiers 83 festgelegt ist. Dagegen ist nun für den gleichen Zweck ein Längsschlitz 84 in dem plattenförmigen Teil 85 des verstellbaren Anschlagorgans 86 vorgesehen, welches längs einer Rippe 87 an der Seitenwange 82 geradlinig geführt und im übrigen gleich ausgebildet ist wie das betreffende Anschlagorgan 73 bei der Ausführungsform nach den Fig. 10 und 11. Eine Klemmplatte 88, in welche der Schraubenbolzen 81 eingeschraubt ist, hält das verstellbare Anschlagorgan 86 in der eingestellten Lage fest. Bei gelöster Klemmverbindung drückt eine z.B. zwischen der Klemmplatte 88 und dem plattenförmigen Teil 85 angreifende Feder 89 das verstellbare Anschlagorgan 86 gegen den festen Anschlag (70 in Fig. 11). Somit erfolgt hier die Einstellbewegung durch Verschwenken des Visiers 83 in die gewünschte Gebrauchslage. Die Tragvorrichtung 60 und die mit dem Stellknopf 62 betätigbaren Einstellmittel zur Verschiebung des Visiers 83 in Sehrichtung sind im übrigen gleich ausgebildet wie bei der Ausführungsform nach den Fig. 10 und 11.

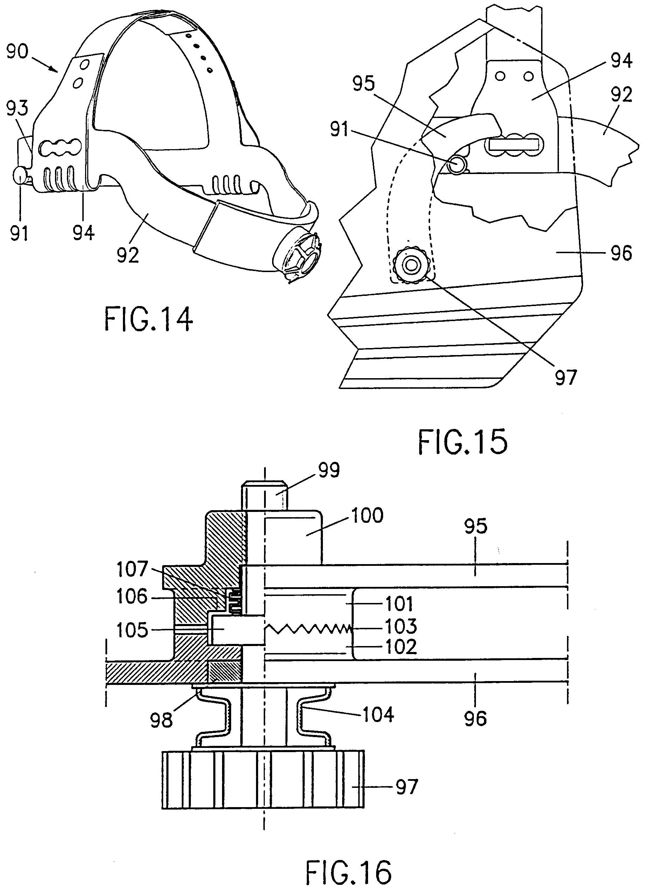

In den Fig. 14 bis 16 ist eine weitere Ausführungsform der Anschlagvorrichtung dargestellt. Diese ist einerseits wiederum so konzipiert, dass sie für die Verstellung des verstellbaren Anschlagorgans keinen Längsschlitz in der Seitenwange des Visiers benötigt. Andererseits stellt sie eine mögliche Lösung dar für den Fall, dass die Ohrenpartie der Tragvorrichtung für die Anbringung eines Ohrenschützers freizuhalten ist.

Wie aus den Fig. 14 und 15 hervorgeht, ist die Tragvorrichtung 90 der Schutzeinrichtung im wesentlichen gleich ausgebildet wie bei der Ausführungsform nach Fig. 8. Im Gegensatz zu dem dortigen Lappen 71, 72 ist hier als fester Anschlag ein runder Zapfen 91 vorgesehen, welcher am Stirnband 92 vor der Vorderkante 93 des abgesetzten Bandabschnittes 94 angeordnet ist und von diesem senkrecht nach aussen ragt. Als verstellbares Anschlagorgan dient ein nach rückwärts gebogener, um die im Bereich des abgesetzten Bandabschnittes 94 liegende Ohrenpartie der Tragvorrichtung 90 herumgeführter Hebel 95, welcher an der Innenseite einer Seitenwange 96 des Visiers an diesem schwenkbar gelagert ist und mit dem Stellknopf 97 in die gewünschte Schwenklage einstellbar ist.

In Fig. 16 ist eine konstruktive Ausgestaltung der Einstellmittel für das verstellbare Anschlagorgan 95 als Beispiel dargestellt. In die Seitenwange des Visiers 96 ist eine Lagerbüchse 98 eingesetzt, in welcher ein Schraubenbolzen 99 drehbar und achsial verschiebbar gelagert ist. Auf dem äusseren Ende des Schraubenbolzens 99 sitzt der Einstellknopf 97, während das innere Ende desselben in eine Nabe 100 des als verstellbares Anschlagorgan dienenden Hebels 95 eingeschraubt ist. An den einander zugewandten Seiten des Hebels 95 und der Seitenwange 96 sind hohlzylindrische Teile 101 bzw. 102 angeformt, welche mit einer gegenseitig zum Eingriff kommenden Kronenverzahnung 103 versehen sind und zusammen eine Rastkupplung bilden. Eine Druckfeder 104, welche zwischen der Seitenwange 96 und dem Stellknopf 97 eingesetzt ist, hält die Rastkupplung geschlossen und damit den Hebel 95 in der eingestellten Lage. An dem Schraubenbolzen 99 befindet sich ein Bund 105, welcher mit einer Schulter des hohlzylindrischen Teils 101 in der Weise zusammenarbeitet, dass durch Lösen der Schraubverbindung anhand des Stellknopfes 97 und achsiales Verschieben des Schraubenbolzens durch Druck auf den Stellknopf 97, entgegen der Wirkung der Druckfeder 104, die Rastkupplung 101, 102, 103 gelöst wird. Ferner ist zwischen dem Hebel 95 und dem Bund 105 am Schraubenbolzen 99 eine als Rutschkupplung wirkende Druckfeder 107 vorgesehen, welche beim Drehen des Stellknopfes 97 den entrasteten Hebel 95 in die gewünschte Anschlagstellung mitnimmt. Beim Loslassen des Stellknopfes 97 fällt unter der Wirkung der Druckfeder 104 die Rastkupplung 101, 102, 103 ein, wobei die Rastwirkung durch anschliessendes Anziehen der Schraubverbindung erhöht werden kann.

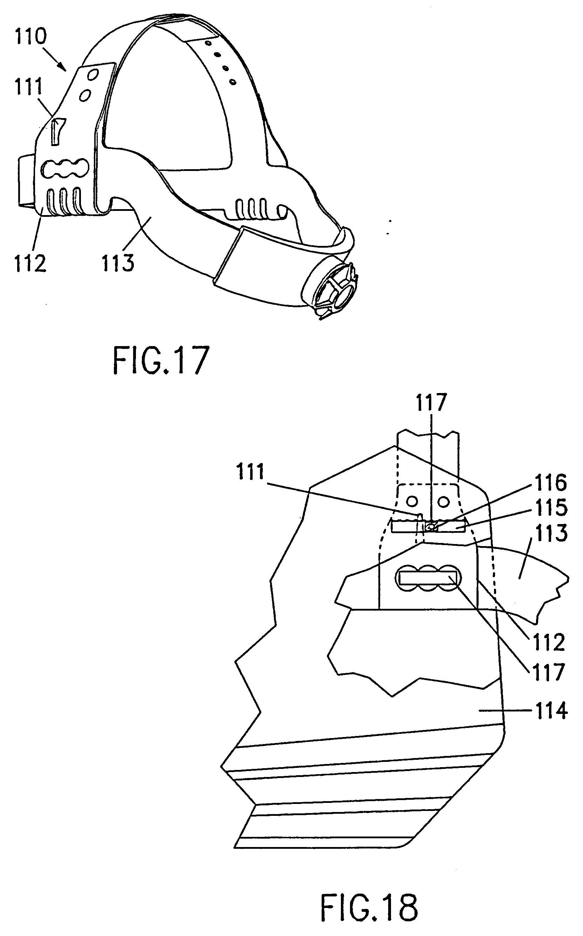

Eine andere, einfachere Ausführungsform der Anschlagvorrichtung, welche ebenfalls eine Freihaltung der Ohrenpartie ermöglicht, ist in den Fig. 17 und 18 dargestellt. An der Tragvorrichtung 110 ist bei diesem Beispiel der feste Anschlag 111 im oberen Bereich des abgesetzten Bandabschnittes 112 des Stirnbandes 113 angeordnet. Auf gleicher Höhe befindet sich in der betreffenden Seitenwange 114 des Visiers ein Längsschlitz 115, in welchem ein verstellbares Anschlagorgan in Form eines Stiftes 116 verschiebbar angeordnet ist, wobei der als Träger dieses Stiftes 116 dienende Lagerkörper 117 und dessen Fixierung mit Hilfe eines Stellknopfes hier nicht näher dargestellt sind. Der Längsschlitz 115 verläuft etwa parallel zum dem Längsschlitz 117, in welchem die Gelenkachse des Visiers verankert ist.

## Patentansprüche

1. Einrichtung zum Schutz des menschlichen Kopfes gegen äussere Einwirkungen, mit einer am Kopf des Trägers der Schutzeinrichtung zu befestigenden, ein steif-elastisches Stirnband aufweisenden Tragvorrichtung (1; 60; 90; 110) und mit einem die Schutzfunktion ausübenden Visier (2; 61; 83), welches mit der Tragvorrichtung aus der Gebrauchslage hochschwenkbar verbunden ist, und mit Einstellmitteln zum Verstellen und Fixieren der Lage des Visiers (2; 61; 83) in Sehrichtung, und mit einem die untere Schwenkendlage des Visiers (2; 61; 83) bildenden Anschlag (50, 73, 86, 95, 117), **dadurch gekennzeichnet**, dass der die untere Schwenkendlage des Visiers (2; 61; 83) bildende Anschlag (50, 73, 86, 95, 117) verstell- und fixierbar ist, dass alle Bedienungselemente (34, 37; 62, 69; 80; 97) der Einstellmittel an der Aussenseite des Visiers (2; 61; 83) der Schutzeinrichtung angeordnet sind, so dass in der Gebrauchslage der Schutzeinrichtung auf einfache Weise die Einstellmittel durch den Träger der Schutzeinrichtung betätigbar und damit die Fixlagen des Visiers veränderbar sind, und dass das Visier (2; 61; 83) unmittelbar am Stirnband (4; 92; 113) angelenkt ist.

2. Schutzeinrichtung nach Anspruch 1, wobei das Visier Seitenwangen (31, 32; 63; 82; 96; 114) aufweist, welche an der Tragvorrichtung (1; 60; 90; 110) angelenkt und durch je eine lösbare, als Rutschkupplung arbeitende Schraubverbindung (34, 40) an dieser gehalten sind, dadurch gekennzeichnet, dass am Stirnband (4; 92; 113) in dessen Schläfenpartien je ein radial nach aussen abgesetzter Bandabschnitt (21; 67; 94; 112) gebildet und als Basisteil für die Festlegung der Schraubverbindung (34, 40) ausgebildet ist.

3. Schutzeinrichtung nach Anspruch 2, dadurch gekennzeichnet, dass der abgesetzte Bandabschnitt (21; 67; 94; 112) und die diesen überdeckende Seitenwange (31, 32; 63; 82; 96; 114) des Visiers (2; 61; 83) je eine Durchgangsöffnung (28, 33; 64, 65; 117) für einen die Schwenkachse des Visiers bildenden Schraubenbolzen (40) aufweisen, wobei eine der beiden Durchgangsöffnungen als Langloch (33; 65; 117) ausgebildet ist, welches wenigstens annähernd parallel zur Sehrichtung verläuft.

4. Schutzeinrichtung nach Anspruch 3, dadurch gekennzeichnet, dass das Langloch (33; 65; 117) in der Längsrichtung einige Raststellen (35; 66) zur wahlweisen Fixierung des Schraubenbolzens (40) durch Festziehen der Schraubverbindung mit Hilfe eines ausserhalb der Seitenwange (31, 32; 63; 96; 114) des Visiers (2; 61; 83) auf den Schraubenbolzen aufgesetzten Stellknopfes (34; 62) aufweist.

5. Schutzeinrichtung nach Anspruch 4, dadurch gekennzeichnet, dass drei Raststellen (35; 66) vorgesehen sind.

6. Schutzeinrichtung nach Anspruch 3, dadurch gekennzeichnet, dass sich die als Langloch ausgebildete Durchgangsöffnung (65; 117) in den abgesetzten Bandabschnitten (67; 94; 112) des Stirnbandes (4; 92; 113) befindet.

7. Schutzeinrichtung nach einem der Ansprüche 2 bis 6, dadurch gekennzeichnet, dass eine Anschlagvorrichtung (29, 50; 70, 73; 86; 91, 95; 111, 117) zum Fixieren der unteren Schwenkendlage des Visiers (2; 61; 83) vorgesehen ist, welche aus einem festen Anschlag und einem mit diesem zusammenarbeitenden verstellbaren Anschlagorgan besteht, wobei der feste Anschlag (29; 70; 91; 111) an einem der abgesetzten Bandabschnitte (21; 67; 94; 112) des Stirnbandes (4; 92; 113) und das verstellbare Anschlagorgan (50; 73; 86; 95; 117) an der Innenseite der diesem Bandabschnitt zugewandten Seite der betreffenden Seitenwange (31; 63; 96; 114) des Visiers (2; 61; 83) angeordnet ist.

8. Schutzeinrichtung nach Anspruch 7, dadurch gekennzeichnet, dass das verstellbare Anschlagorgan (50; 73; 86; 117) längs einer geradlinigen Bahn (36, 38; 68, 77; 87; 115) verschiebbar ist und eine quer zur Verschiebebahn verlaufende Anschlagkante (56; 75) aufweist.

9. Schutzeinrichtung nach Anspruch 8 oder 9, dadurch gekennzeichnet, dass das verstellbare Anschlagorgan (50; 73; 86; 95; 117) an der Seitenwange (31; 63; 96; 114) des Visiers (2; 61; 83) durch eine lösbare Schraubverbindung (37, 52; 69; 77; 80, 81; 97, 99) fixiert ist, welche einen die Seitenwange in einer Durchgangsöffnung (36; 68; 115) durchsetzenden Schraubenbolzen (52; 77; 81; 99) und einen an der Aussenseite der Seitenwange auf den Schraubenbolzen aufgesetzten Stellknopf (37; 69; 80; 97) aufweist.

10. Schutzeinrichtung nach Anspruch 9 dadurch gekennzeichnet, dass das verstellbare Anschlagorgan (50) mit dem Schraubenbolzen (52) fest verbunden ist und dass die betreffende Seitenwange (31) des Visiers (2) ein Langloch (36) aufweist, längs welchem der Schraubenbolzen (52) stufenlos verschiebbar und verdrehungssicher gelagert ist (Fig. 6, 7).

11. Schutzeinrichtung nach Anspruch 9, dadurch gekennzeichnet, dass die Lage des Schraubenbolzens (81) in der Durchgangsöffnung der Seitenwange (82) des Visiers festgelegt ist und dass das verstellbare Anschlagorgan (86) ein Langloch (84) aufweist, mit welchem das Anschlagorgan auf dem Schraubenbolzen stufenlos längsverschiebbar und verdrehungssicher gelagert ist, wobei am verschiebbaren Anschlagorgan (86) Federmittel (89) wirksam sind, welche bei gelöster Fixierung das verschiebbare Anschlagorgan (86) gegen den festen Anschlag (70) halten.

12. Schutzeinrichtung nach Anspruch 9, dadurch gekennzeichnet, dass die Lage des Schraubenbolzens (99) in der Durchgangsöffnung der Seitenwange (96) des Visiers festgelegt ist und dass das verstellbare Anschlagorgan (95) durch eine Rutschkupplung (107) mit dem Schraubenbolzen (99) und durch eine durch Achsialverschiebung des Schraubenbolzens (99) ausrückbare Rastkupplung (101, 102) mit der Seitenwange (96) verbunden ist.

13. Schutzeinrichtung nach einem der Ansprüche 7 bis 12, dadurch gekennzeichnet, dass sich die Anschlagvorrichtung (91, 95; 111, 117) und deren Einstellmittel (97, 99; 116) ausserhalb der Ohrenpartie der Tragvorrichtung (90; 110) befinden.

14. Schutzeinrichtung nach Anspruch 1, mit Einstellmitteln zur Anpassung der Tragvorrichtung (1) an den Kopf des Trägers, dadurch gekennzeichnet, dass in der Gebrauchslage der Schutzeinrichtung betätigbare, selbsthemmend wirkende Einstellmittel (13, 16) zur Verstellung des Umfangs des Stirnbandes (4) vorgesehen sind.

15. Schutzeinrichtung nach Anspruch 14, dadurch gekennzeichnet, dass sich die genannten Einstellmittel (13, 16) zur Verstellung des Stirnbandes (4) an der Nackenpartie (15) desselben befinden.

16. Schutzeinrichtung nach Anspruch 14, dadurch gekennzeichnet, dass die freien Enden (7, 8) eines das Stirnband (4) bildenden Bandstücks (6) je ein in Bandlängsrichtung verlaufendes Langloch (11) aufweisen, dessen eine Längskante mit einer Verzahnung (12) versehen ist, wobei die Verzahnungen (12) der beiden Langlöcher (11) auf verschiedenen Längsseiten der Langlöcher liegen, so dass sie bei übereinandergelegten Bandenden (7, 8) einander gegenüberliegen, und dass eine die beiden Bandenden (7, 8) umfassende Schliesse (13) vorgesehen ist, welche im Innern ein in die Verzahnungen der beiden Bandenden eingreifendes Zahnrad sowie eine die Drehung des Zahnrads hemmende Rutschkupplung und aussen einen mit der Achse des Zahnrads verbundenen Drehknopf (14) aufweist.

## Claims

1. Device for protecting the human head against external influences, with a supporting device (1; 60; 90; 110) to be secured on the head of the wearer of the protective device and having a relatively inflexible headstrap, and with a visor (2; 61; 83) which performs the protective function and which is connected to the supporting device in such a way that it can be pivoted up out of the position of use, and with setting means for adjusting and fixing the position of the visor (2; 61; 83) in the direction of sight, and with a stop (50, 73, 86, 95, 117) which forms the lower pivoting end position of the visor (2; 61; 83), characterized in that the stop (50, 73, 86, 95, 117) which forms the lower pivoting end position of the visor (2; 61; 83) is adjustable and fixable; in that all the operating elements (34, 37; 62, 69; 80; 97) of the setting means are arranged on the outside of the visor (2; 61; 83) of the protective device, making it a simple matter, in the position of use of the protective device, for the setting means to be actuated by the wearer of the protective device and hence for the fixed positions of the visor to be changed; and in that the visor (2; 61; 83) is hinged directly on the headstrap (4; 92; 113).

2. Protective device according to Claim 1, the visor having side pieces (31, 32; 63; 82; 96; 114) which are hinged on the supporting device (1; 60; 90; 110) and are held on the latter by respective releasable screwed joints (34, 40) acting as slipping clutches, characterized in that respective strap sections (21; 67; 94; 112) offset radially outwards are formed on the headstrap (4; 92; 113) in the temple portions of the latter and are designed as basic parts for the fixing of the screwed joint (34, 40).

3. Protective device according to Claim 2, characterized in that the offset strap section (21; 67; 94; 112) and the side piece (31, 32; 63; 82; 96; 114), covering the latter, of the visor (2; 61; 83) each have a through opening (28, 33; 64, 65; 117) for a bolt (40) which forms the pivot of the visor, one of the two through openings being designed as an elongate hole (33; 65; 117) which runs at least approximately parallel to the direction of sight.

4. Protective device according to Claim 3, characterized in that the elongate hole (33; 65; 117) has in the longitudinal direction a number of dwell locations (35; 66) for the optional fixing of the bolt (40) by tightening the screwed joint with the aid of a setting knob (34; 62) mounted on the bolt to the outside of the side piece (31, 32; 63; 96; 114) of the visor (2; 61; 83).

5. Protective device according to Claim 4, characterized in that three dwell locations (35; 66) are provided.

6. Protective device according to Claim 3, characterized in that the through opening (65; 117) designed as an elongate hole is situated in the offset strap sections (67; 94; 112) of the headstrap (4; 92; 113).

7. Protective device according to one of Claims 2 to 6, characterized in that a stop device (29, 50; 70, 73; 86; 91, 95; 111, 117) for fixing the lower pivoting end position of the visor (2; 61; 83) is provided which comprises a fixed stop and an adjustable stop member which interacts with the latter, the fixed stop (29; 70; 91; 111) being arranged on one of the offset strap sections (21; 67; 94; 112) of the headstrap (4; 92; 113) and the adjustable stop member (50; 73; 86; 95; 117) being arranged on the inside of that side of the relevant side piece (31; 63; 96; 114) of the visor (2; 61; 83) which faces this strap section.

8. Protective device according to Claim 7, characterized in that the adjustable stop member (50; 73; 86; 117) is displaceable along a rectilinear track (36, 38; 68, 77; 87; 115) and has a stop edge (56; 75) running transversely to the displacement track.

9. Protective device according to Claim 8 or 9, characterized in that the adjustable stop member (50; 73; 86; 95; 117) is fixed on the side piece (31; 63; 96; 114) of the visor (2; 61; 83) by means of a releasable screwed joint (37, 52; 69; 77; 80, 81; 97, 99) which has a bolt (52; 77; 81; 99) that passes through the side piece in a through opening (36; 68; 115), and a setting knob (37; 69; 80; 97) which is mounted on the bolt at the outside of the side piece.

10. Protective device according to Claim 9, characterized in that the adjustable stop member (50) is rigidly connected to the bolt (52) and in that the relevant side piece (31) of the visor (2) has an elongate hole (36) along which the bolt (52) can be displaced in an infinitely variable manner and is mounted in a manner secure against rotation (Figs. 6, 7).

11. Protective device according to Claim 9, characterized in that the position of the bolt (81) in the through opening in the side piece (82) of the visor is fixed and in that the adjustable stop member (86) has an elongate hole (84) by means of which the stop member can be displaced longitudinally in an infinitely variable manner on the bolt and is mounted in a manner secure against rotation, spring means (89) which, with the fixing released, hold the displaceable stop member (86) against the fixed stop (70), acting on the displaceable stop member (86).

12. Protective device according to Claim 9, characterized in that the position of the bolt (99) in the through opening in the side piece (96) of the visor is fixed and in that the adjustable stop member (95) is connected to the bolt (99) by means of a slipping clutch (107) and to the side piece (96) by means of a locking clutch (101, 102) which can be disengaged by axial displacement of the bolt (99).

13. Protective device according to one of Claims 7 to 12, characterized in that the stop device (91, 95; 111, 117) and its setting means (97, 99; 116) are situated to the outside of the ear portion of the supporting device (90; 110).

14. Protective device according to Claim 1, with setting means for adapting the supporting device (1) to the head of the wearer, characterized in that setting means (13, 16) with a self-locking action that can be actuated in the position of use of the protective device are provided for the adjustment of the circumference of the headstrap (4).

15. Protective device according to Claim 14, characterized in that the said setting means (13, 16) for adjusting the headstrap (4) are situated on the neck portion (15) of the latter.

16. Protective device according to Claim 14, characterized in that the free ends (7, 8) of a piece of strap (6) forming the headstrap (4) each have an elongate hole (11) which runs in the longitudinal direction of the strap and one longitudinal edge of which is provided with teeth (12), the teeth (12) of the two elongate holes (11) being situated on different longitudinal sides of the elongate holes so that they lie opposite one another when the ends (7, 8) of the strap are laid one on top of the other; and in that a clasp (13) which encloses the two ends (7, 8) of the strap is provided which, on the inside, has a toothed wheel which engages in the teeth of both ends of the strap, and a slipping clutch which inhibits the rotation of the toothed wheel, and, on the outside, has a rotary knob (14) connected to the spindle of the toothed wheel.

## Revendications

1. Dispositif pour la protection de le tête humaine contre des effets extérieurs, équipé d'un dispositif de port (1; 60; 90; 110) à fixer sur la tête du porteur du dispositif de protection et présentant un bandeau rigide et élastique et d'une visière (2; 61; 83) assumant la fonction de protection qui est reliée au dispositif de port de façon à pouvoir pivoter à partir de la position d'utilisation, et de moyens d'ajustage pour le réglage et la fixation de la position de la visière (2; 61; 83) dans le sens du regard, et d'une butée (50, 73, 86, 95, 117) formant la position finale inférieure de pivotement de la visière (2; 61; 83), caractérisé en ce que la butée (50, 73, 86, 95, 117) formant la position finale inférieure de pivotement de la visière (2; 61; 83) peut être rélée et fixée, en ce que tous les éléments de commande (34, 37; 62, 69; 80; 97) des moyens de réglage sont disposés sur le côté extérieur de le visière (2; 61; 83) du dispositif de protection, de sorte que les moyens de réglage peuvent être actionnés de façon simple par le porteur du dispositif de protection et les positions fixes de la visière peuvent donc être modifiées dans la position d'utilisation du dispositif de protection, et en ce que la visière (2; 61; 83) est articulée directement sur le bandeau (4; 92; 113).

2. Dispositif de protection selon la revendication 1, la visière présentant des joues latérales (31, 32; 63; 82; 96; 114) qui sont articulées sur le dispositif de port (1; 60; 90; 110) et sont maintenues sut celui-ci par un assemblage vissé (34, 40) amovible et fonctionnant comme un accouplement patinant, caractérisé en ce qu'une partie de ruban (21; 67; 94; 112) décalée radialement vers l'extérieur est formée sur le bandeau (4; 92; 113) dans chacune de ses parties temporales et est réalisée comme partie de base pour la fixation de l'assemblage vissé (34, 40).

3. Dispositif de protection selon la revendication 2, caractérisé en ce que la partie de ruban (21; 67; 94; 112) décalée et la joue latérale (31, 32; 63; 82; 96; 114), recouvrant celle-ci, de la visière (2; 61; 83) présentent chacune un orifice de passage (28, 33; 64, 65; 117) pour un boulon fileté (40) formant l'axe de pivotement de la visière, l'un des deux orifices de passage se présentant sous la forme d'un trou oblong (33; 65; 117) qui est au moins approximativement parallèle à la direction du regard.

4. Dispositif de protection selon la revendication 3, caractérisé en ce que le trou oblong (33; 65; 117) présente dans le sens longitudinal quelques positions de crantage (35; 66) pour la fixation optionnelle du boulon fileté (40) par le blocage de l'assemblage vissé à l'aide d'un bouton de réglage (34; 62) posé sur le boulon fileté à l'extérieur de la joue latérale (31, 32; 63; 96; 114) de la visière (2; 61; 83).

5. Dispositif de protection selon la revendication 4, caractérisé en que trois positions de crantage (35; 66) sont prévues.

6. Dispositif de protection selon la revendication 3, caractérisé en ce que l'orifice de passage (65; 117) conçu comme un trou oblong se trouve dans les parties de ruban (67; 94; 112) décalées du bandeau (4; 92; 113).

7. Dispositif de protection selon l'un quelconque des revendications 2 à 6, caractérisé en ce qu'un dispositif de butée (29, 50; 70, 73; 86; 91, 95; 111, 117) est prévu pour la fixation de la position finale inférieure de pivotement de la visière (2; 61; 83), lequel dispositif comprend une butée fixe et un organe de butée réglable et travaillant conjointement avec la première, la butée fixe (29; 70; 91; 111) étant disposée sur l'une des parties de ruban (21; 67; 94; 112) décalées du bandeau (4; 92; 113) et l'organe de butée (50; 73; 86; 95; 117) réglable sur la face intérieure du côté, tourné vers cette partie de bandeau, de la joue latérale (31; 63; 96; 114) concernée de la visière (2; 61; 83)

8. Dispositif de protection selon la revendication 7, caractérisé en ce que l'organe de butée (50; 73; 86; 117) réglable peut être déplacé le long d'une trajectoire (36, 38; 68; 77, 87; 115) rectiligne et présente une arête de butée (56; 75) transversale à la trajectoire de coulissement.

9. Dispositif de protection selon la revendication 8 ou 9, caractérisé en ce que l'organe de butée (50; 73; 86; 95; 117) réglable est fixé sur la joue latérale (31; 63; 96; 114) de la visière (2; 61; 83) par un assemblage vissé (37, 52; 69, 77; 80, 81; 97, 99) amovible qui présente un boulon fileté (52; 77; 81; 99) traversant la joue latérale dans un orifice de passage (36; 68; 115) et un bouton de réglage (37; 69; 80; 97) posé sur le boulon fileté sur le côté extérieur de la joue latérale.

10. Dispositif de protection selon la revendication 9, caractérisé en ce que l'organe de butée (50) réglable est relié de façon fixe au boulon fileté (52) et en ce que la joue latérale (31) concernée de la visière présente un trou oblong (36), le long duquel le boulon fileté (52) peut être déplacé de façon progressive et est logé de façon à résister à la torsion (figures 6 et 7).

11. Dispositif de protection selon la revendication 9, caractérisé en ce que la position du boulon fileté (81) est fixée dans l'orifice de passage de la joue latérale (82) de la visière et en ce que l'organe de butée (86) réglable présente un trou oblong (84), avec lequel l'organe de butée peut être déplacé dans le sens longitudinal progressivement sur le boulon fileté et est logé de façon à résister à la torsion, des ressorts (89) étant efficaces sur l'organe de butée (86) pouvant coulisser, lesquels ressorts maintiennent l'organe de butée (86) coulissante contre la butée (70) fixe lorsque la fixation est enlevée.

12. Dispositif de protection selon la revendication 9, caractérisé en ce que la position du bouton fileté (99) est définie dans l'orifice de passage de la joue latérale (96) de la visière et en ce que l'organe de butée (95) réglable est relié au boulon fileté (99) par un accouplement patinant (107) et à la joue latérale (96) par un accouplement patinant (101, 102) débrayable par déplacement axial du boulon fileté (101, 102).

13. Dispositif de protection selon l'une quelconque des revendications 7 à 12, caractérisé en ce que le dispositif de butée (91, 95; 111, 117) et ses moyens de réglage (97, 99; 116) se trouvent en dehors de la partie des oreilles du dispositif de port (90; 110).

14. Dispositif de protection selon la revendication 1, avec des moyens de réglage pour l'adaptation du dispositif de port (1) à la tête du porteur, caractérisé en ce que des moyens de réglage (13, 16) actionnables dans la position d'utilisation du disposition de protection et à effet autobloquant sont prévus pour le réglage de la circonférence du bandeau (4).

15. Dispositif de protection selon la revendication 14, caractérisé en ce que les moyens de réglage (13, 16) cités pour le réglage du bandeau (4) se trouvent sur la partie nuque de celui-ci.

16. Dispositif de protection selon la partie 14, caractérisé en ce que les extrémités (7, 8) libres d'une partie du ruban (6) formant le bandeau (4) présentent chacune un trou oblong (11) allant dans le sens longitudinal du ruban, dont une arête longitudinale est pourvue d'une denture (12), les dentures (12) des deux trous oblongs (11) étant situées sur différents grands côtés des trous oblongs, de sorte qu'elles se font face lorsque les extrémités du ruban (7, 8) sont superposées, et en ce qu'on prévoit une fermeture (13) comprenant les deux extrémités de bande (7,8), laquelle présente à l'intérieur une roue dentée s'engageant dans les dentures des deux extrémités du ruban et un accouplement patinant empêchant la rotation de la roue dentée et à l'extérieur un bouton tournant (14) relié à l'axe de la roue dentée.
